# EUROPEAN PATENT APPLICATION

(11) **EP 4 814 010 A1**
(43) Date of publication of application: **30.09.2026**
(21) Application number: 24893499.4
(22) Date of filing: 21.11.2024
(51) Int. Cl.: C07K 16/24, A61P 35/00, A61K 39/00

(54) **ANTIGEN BINDING PROTEIN TARGETING IL-11**

(30) Priority: 22.11.2023 CN 202311567491
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: YU, Haijia, Shanghai 201203 (CN); ZHAN, Yifan, Shanghai 201203 (CN); REN, Xiaochen, Shanghai 201203 (CN); PENG, Guoyuan, Shanghai 201203 (CN); LIAO, Jiang, Shanghai 201203 (CN); ZHU, Xiangyang, Shanghai 201203 (CN)
(74) Representative: Weickmann & Weickmann PartmbB
(86) International application number: PCT/CN2024/133382
(87) International publication number: WO 2025/108343

(57) **Abstract**

The present application relates to an isolated antigen-binding protein which targets IL-11. The present application further provides a preparation method and use of the isolated antigen-binding protein.

## Description

### TECHNCIAL FIELD

The present application relates to the field of biomedicines, and specifically relates to an isolated antigen-binding protein targeting IL-11 and use thereof.

### BACKGROUND ART

Interleukin-11 is a cytokine derived from stromal cells, and it belongs to the IL-6 superfamily, is secreted by various types of cells such as osteoblasts, synovial cells, fibroblasts, chondrocytes, and trophoblasts, then is bound to IL-11R alpha and GP130 receptors and activates a downstream JAK/STAT signaling pathway.

In a growing number of researches, IL-11 has been found to play an extremely important role in the fields of cell protection, anti-inflammatory, anti-fibrosis and the like of organs such as the lung. IL-11 is secreted by post-injury polarized cells or fibroblasts and has autocrine and paracrine effects. In the polarized cells, IL-11 causes cellular dysfunction, initiates apoptotic cell death, and blocks regeneration at the same time. In stromal cells, IL-11 triggers the extracellular matrix production, invasion, and migration of myofibroblasts. The fibroblasts and myofibroblasts activated by IL-11 can secrete cytokines and chemokines, which have high pro-inflammatory effect. Inhibiting the IL-11 can protect parenchyma, resist fibrosis, and alleviate matrix-driven inflammation. In regenerative tissues, inhibiting the IL-11 can promote the proliferation and regeneration of injured cells (such as hepatocytes) and organ regeneration.

In early 2020, Boehringer Ingelheim announced to acquire the global exclusive rights to preclinical IL-11 platform of Enleofen, and developed monoclonal antibody drugs similar in targeting IL-11/IL-11Ra, so as to develop first-in-class therapies for treating various fibroinflammatory diseases. A monoclonal antibody drug LASN01 targeting IL-11Ra developed by Lassen Therapeutics was expected to be used as a new treatment method for fibrosis and neoplastic cancers, and an investigational new drug application was submitted to the FDA in 2021 for initiating a Phase I clinical trial. In China, an IL-11 monoclonal antibody (9MW3811) developed by Mabwell Bioscience is in the lead, a clinical trial application was submitted to the FDA in February 2023, and it is currently in the Phase I clinical stage.

As described above, there are a small number of relevant researches taking IL-11 as a target at present, and all are in the early research stage of development, and no related drugs have been approved for marketing, so the drugs targeting this target has broad clinical application and therapeutic prospects.

### SUMMARY

The present application provides an isolated antigen-binding protein, which targets IL-11 and has the following advantages: (1) the affinity to human and/or animal IL-11 is extremely high; (2) the activation of an IL-11-mediated hIL-11 Effector Reporter Cell can be blocked; and (3) fibrosis can be inhibited.

In a first aspect, the present application provides an isolated antigen-binding protein, which contains at least one CDR in a heavy chain variable region VH or VHH.

In some embodiments, the antigen-binding protein contains HCDR3, and an amino acid sequence of the HCDR3 is as set forth in SEQ ID NO: 3.

In some embodiments, the antigen-binding protein contains HCDR2, and an amino acid sequence of the HCDR2 is as set forth in SEQ ID NO: 2.

In some embodiments, the antigen-binding protein contains HCDR1, and an amino acid sequence of the HCDR1 is as set forth in SEQ ID NO: 1.

In some embodiments, the antigen-binding protein contains HCDR3, HCDR2 and HCDR1; and the amino acid sequence of the HCDR3 is as set forth in SEQ ID NO: 3, the amino acid sequence of the HCDR2 is as set forth in SEQ ID NO: 2, and the amino acid sequence of the HCDR1 is as set forth in SEQ ID NO: 1.

In some embodiments, the antigen-binding protein contains H-FR1; and a C terminal of the H-FR1 is directly or indirectly linked to an N terminal of the HCDR1, and an amino acid sequence of the H-FR1 is as set forth in SEQ ID NO: 4.

In some embodiments, the antigen-binding protein contains H-FR2; and the H-FR2 is located between the HCDR1 and the HCDR2, and an amino acid sequence of the H-FR2 is as set forth in SEQ ID NO: 5.

In some embodiments, the antigen-binding protein contains H-FR3; and the H-FR3 is located between the HCDR2 and the HCDR3, and an amino acid sequence of the H-FR3 is as set forth in SEQ ID NO: 6.

In some embodiments, the antigen-binding protein contains H-FR4; and an N terminal of the H-FR4 is linked to a C terminal of the HCDR3, and an amino acid sequence of the H-FR4 is as set forth in SEQ ID NO: 7.

In some embodiments, the antigen-binding protein contains H-FR1, H-FR2, H-FR3 and H-FR4; and the amino acid sequence of the H-FR1 is as set forth in SEQ ID NO: 4, the amino acid sequence of the H-FR2 is as set forth in SEQ ID NO: 5, the amino acid sequence of the H-FR3 is as set forth in SEQ ID NO: 6, and the amino acid sequence of the H-FR4 is as set forth in SEQ ID NO: 7.

In some embodiments, the antigen-binding protein contains an antibody heavy chain variable region VH or VHH, and the amino acid sequence of the VH or VHH is as set forth in SEQ ID NO: 10.

In some embodiments, the antigen-binding protein contains an antibody or an antigen-binding fragment thereof.

In some embodiments, the antigen-binding protein includes Fab, Fab', F(ab)₂, a Fv fragment, F(ab')₂, scFv, di-scFv, VHH and/or dAb.

In some embodiments, the antibody is selected from the group consisting of a monoclonal antibody, a chimeric antibody, a humanized antibody, a fully human antibody, a nanobody and/or a heavy chain antibody.

In some embodiments, the antibody is a nanobody and/or a heavy chain antibody.

In some embodiments, the antigen-binding fragment is VHH.

In some embodiments, the amino acid sequence of the VHH is as set forth in SEQ ID NO: 10.

In some embodiments, the antigen-binding protein contains an antibody heavy chain constant region.

In some embodiments, the amino acid sequence in the antibody heavy chain constant region is as set forth in SEQ ID NO: 8 or SEQ ID NO: 9.

In another aspect, the present application further provides a chimeric antigen receptor, which contains a targeting domain, and the targeting domain contains the antigen-binding protein.

In another aspect, the present application further provides polypeptide, which contains the antigen-binding protein.

In another aspect, the present application further provides an immunoconjugate, which contains the isolated antigen-binding protein, and/or the polypeptide.

In another aspect, the present application further provides one or more isolated nucleic acid molecules, which encode the isolated antigen-binding protein and/or the chimeric antigen receptor.

In another aspect, the present application further provides a vector, which contains the nucleic acid molecules.

In another aspect, the present application further provides a cell, which contains the antigen-binding protein, the chimeric antigen receptor, the polypeptide, the immunoconjugate, the nucleic acid molecules and/or the vector.

In another aspect, the present application further provides a method for preparing the antigen-binding protein, the chimeric antigen receptor and the polypeptide, which includes: culturing the cell under conditions that allow expression of the antigen-binding protein, the polypeptide and the chimeric antigen receptor.

In another aspect, the present application further provides a method for preparing modified immune cells, which includes: introducing the vector into the immune cells.

In another aspect, the present application further provides a pharmaceutical composition, which contains the antigen-binding protein, the chimeric antigen receptor, the polypeptide, the immunoconjugate, the nucleic acid molecules, the vector, the cell, and/or a pharmaceutically acceptable carrier.

In another aspect, the present application further provides a method for detecting presence and/or content of IL-11 protein in a sample, which includes: using the antigen-binding protein, the chimeric antigen receptor, the polypeptide, the immunoconjugate, the nucleic acid molecules, the vector, the cell, and/or the pharmaceutical composition.

In another aspect, the present application further provides a kit for detecting IL-11, which contains the antigen-binding protein, the chimeric antigen receptor, the polypeptide, the immunoconjugate, the nucleic acid molecules, the vector, the cell, and/or the pharmaceutical composition.

In another aspect, the present application further provides use of the antigen-binding protein, the chimeric antigen receptor, the polypeptide, and/or the cell in preparation of the kit.

In another aspect, the present application further provides use of the antigen-binding protein, the chimeric antigen receptor, the polypeptide, the immunoconjugate, the cell, and/or the pharmaceutical composition in preventing, alleviating and/or treating diseases and/or disorders. In another aspect, the present application further provides use of the antigen-binding protein, the chimeric antigen receptor, the polypeptide, the immunoconjugate, the cell, and/or the pharmaceutical composition in the manufacture of a medicament for preventing and/or treating diseases and/or disorders.

In some embodiments, the drugs are used for preventing, alleviating and/or treating diseases and/or disorders.

In some embodiments, the diseases and/or disorders include diseases associated to IL-11.

In some embodiments, the diseases and/or disorders include inflammatory disease or tumor.

In some embodiments, the diseases and/or disorders include inflammatory disease and/or tumor associated to IL-11.

Those skilled in the art can easily discern other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
FIG. 1 shows an activation effect curve of an anti-IL-11 antibody in inhibiting an IL-11-mediated hIL-11 Effector Reporter Cell according to the present application.
FIG. 2 shows a PCR result of an ability of an anti-IL-11 antibody in inhibiting TGFβ-induced fibrosis in NICH0018 (skin fibroblasts of healthy humans) according to the present application.
FIG. 3 shows an ELISA result of an ability of an anti-IL-11 antibody in inhibiting TGFβ-induced fibrosis in NICH0018 (skin fibroblasts of healthy humans) according to the present application.

### DETAILED DESCRIPTION

The embodiments of the invention of the present application will be described below with specific examples. Those skilled in the art can easily understand other advantages and effects of the invention of the present application from the disclosure of the specification.

### Definition of Terms

In the present application, the term "IL-11" generally refers to a cytokine derived from stromal cells and belongs to the IL-6 superfamily. The "IL-11" covers "full-length", unprocessed IL-11 and any form of IL-11 produced by cellular processing. In the present application, the term "IL-11" includes its mutants, fragments, variants, isoforms, and homologs.

In the present application, the term "isolated" usually refers to collecting from a natural state by an artificial means. If an "isolated" substance or component occurs in nature, it may be that the natural environment in which the substance or component is has changed, or that the substance has been isolated from the natural environment, or both. For example, a certain unisolated polynucleotide or polypeptide naturally exists in a living animal, and the same polynucleotide or polypeptide with high purity isolated from the natural state is called "isolated". The term "isolated" does not exclude the presence of artificial or synthetic substances, nor other impure substances that do not affect the activity of the substances.

In the present application, the term "isolated antigen-binding protein" typically refers to a protein which is collected from a natural state by an artificial means and has an antigen-binding ability. The "isolated antigen-binding protein" may include an antigen-binding domain, and optionally, a framework or frame region that allows the antigen-binding moiety to use a conformation that promotes the antigen-binding domain to bind to an antigen. The antigen-binding protein may comprise, for example, antibody-derived protein framework regions (FRs) or alternative protein framework regions or artificial framework regions with grafted CDRs or CDR derivatives. Such frameworks include, but are not limited to, an antibody-derived framework region including a mutation introduced, for example, to stabilize a three-dimensional structure of the antigen-binding protein, and a fully synthetic framework region containing, for example, a biocompatible polymer. Examples of the antigen-binding protein include, but are not limited to: a human antibody, a humanized antibody, a chimeric antibody, a recombinant antibody, a single-chain antibody, a bifunctional antibody, a trifunctional antibody, a four-function antibody, Fab, Fab', a Fv fragment, F(ab')₂, F(ab)₂, scFv, di-scFv, dAb, an IgD antibody, an IgE antibody, an IgM antibody, an IgG1 antibody, an IgG2 antibody, an IgG3 antibody, or an IgG4 antibody and a fragment thereof.

In the present application, the term "CDR" is also known as "complementarity-determining region", and generally refers to a region in an antibody variable domain, and its sequence is hypervariable and/or forms a structurally defined loop. Generally, the antibody includes six CDRs, three (HCDR1, HCDR2, HCDR3) in VH, and three (LCDR1, LCDR2, LCDR3) in VL. In some embodiments, natural camel antibodies consisting only of heavy chains have normal and stable functions in the absence of light chains. The antibody CDR can be determined by a variety of encoding systems, such as CCG, Kabat, Chothia, IMGT, and Kabat/Chothia integrated into account. The encoding systems are known in the art. For example, the amino acid sequence number of the antigen-binding protein can be divided according to an IMGT numbering system. For example, the CDR of the antigen-binding protein can be determined according to a Kabat system.

In the present application, the term "FR" generally refers to a more highly conserved portion of an antibody variable domain, which is referred to as a framework region. Generally, the variable domains of natural heavy chains and light chains each contains four FR regions, namely, four (H-FR1, H-FR2, H-FR3 and H-FR4) in VH, and four (L-FR1, L-FR2, L-FR3 and L-FR4) in VL.

In the present application, the term "variable domain" and "variable region" are interchangeably used with each other, and generally refer to a part of antibody heavy chains/or light chains. Variable domains of heavy and light chains can be referred to as "V_{H}" and "V_{L}" (or "VH" and "VL"), respectively. The domains are generally the most variable part of the antibody (relative to other antibodies of the same type), and contains antigen binding sites.

In the present application, the term "VHH" is interchangeably used with "heavy chain antibody" and generally refers to an antibody that contains a variable antigen-binding domain of the heavy chain antibody. VHH may also be referred to as a nanobody (Nb) and/or single-region antibody. For example, the VHH can bind to IL-11. For example, the VHH has specificity for IL-11.

In the present application, the term "chimeric antigen receptor" (CAR) generally refers to a recombinant polypeptide that contains at least extracellular domains, transmembrane regions, and intracellular domains that specifically bind to an antigen or target. For example, a hinge region is included between the extracellular domain and the transmembrane region. For example, the chimeric antigen receptor may include a signal peptide. The extracellular domain of the CAR binds to the target antigen on the surface of a target cell, resulting in clustering of CAR and the delivery of activation stimulus to CAR-containing cells. CAR redirects the specificity of immunologic effector cells and triggers proliferation, cytokine production, and mediates phagocytosis and/or production of molecules that induce cell death of target antigen in a manner independent of major histocompatibility (MHC). For example, the extracellular structure may include the above antigen-binding protein. For example, the extracellular structures can specifically bind to the IL-11.

In the present application, the terms "polypeptide molecule", "polypeptide" and "peptide" are interchangeably used and generally refer to polymers of amino acid residues. The term "fusion protein" usually refers to a polypeptide that has at least two parts covalently linked together. Each part may be polypeptides with different properties. This property can be of a biological nature, such as in vitro or in vivo activity. The property can also be a simple chemical or physical property, such as binding to a target molecule, catalysis of a reaction. The two parts can be linked directly by a single peptide bond or by a peptide linker.

In the present application, the term "nucleic acid molecules" generally refers to nucleotides, deoxyribonucleotides or ribonucleotides in an isolated form of any length, or analogues isolated from natural environment or artificially synthesized.

In the present application, the term "vector" generally refers to a nucleic acid carrying tool into which polynucleotide encoding a certain protein can be inserted and the protein is expressed. A vector may be transformed, transduced, or transfected into a host cell to express a genetic material element it carries within the host cell. For example, vectors may include plasmids; phagemids; Cosmids; artificial chromosomes such as yeast artificial chromosomes (YAC), bacterial artificial chromosomes (BAC), or P1 derived artificial chromosomes (PAC); phages such as λ phage or M13 phage; animal viruses; and the like. The types of animal viruses used as vectors include retroviruses (including lentiviruses), adenoviruses, adeno-associated viruses, herpes viruses (e.g., herpes simplex virus), poxviruses, baculoviruses, papillomaviruses, and papovaviruses (e.g., SV40). A vector may include a variety of elements that control expression, including a promoter sequence, a transcription initiation sequence, an enhancer sequence, a selection element, and a reporter gene. In addition, the vector may also contain a replication initiation site. The vector may also include components that facilitate to enter cells, such as viral particles, liposomes, or protein coats, but not just these.

In the present application, the term "cell" generally refers to a single cell, cell line, or cell culture that may be, or has been a recipient of a subject plasmid or vector, which includes a nucleic acid molecule according to the present application or the vector according to the present application. The cell can include the progeny of a single cell. Due to natural, accidental or intentional mutations, the progeny may not necessarily be identical to the original mother cell (in the morphology or genome of the total DNA complement). The cell may include a cell transfected in vitro with the vector according to the present application. Cells can be bacterial cells (e.g., Escherichia coli), yeast cells, or other eukaryotic cells, such as COS cells, Chinese hamster ovary (CHO) cells, CHO-K1 cells, LNCAP cells, HeLa cells, HEK293 cells, COS-1 cells, NS0 cells. In some embodiments, the cells are mammalian cells. In some embodiments, mammalian cells are HEK293 cells.

In the present application, the term "immunoconjugate" generally refers to a conjugate formed by the conjugation (e.g., by covalent linkage through joint molecules) of other agents (for example, chemotherapeutic agents, radioactive elements, cell growth inhibitors, and cytotoxic agents) to the antibody or antigen-binding fragment thereof, and the conjugate can deliver other agents to the target cells (e.g., tumor cells) by the antibody or antigen-binding fragment thereof specifically binding to the antigen on the target cells.

In the present application, the term "pharmaceutical composition" generally refers to compositions for preventing/treating diseases or disorders. The pharmaceutical composition may comprise the isolated antigen-binding protein according to the present application, the nucleic acid molecule according to the present application, the vector according to the present application, and/or the cell according to the present application, and optionally a pharmaceutically acceptable adjuvant. In addition, the pharmaceutical composition may further comprise suitable preparations of one or more (pharmaceutically effective) carriers, stabilizers, excipients, diluents, solubilizers, surfactants, emulsifiers and/or preservatives. Acceptable ingredients of the composition are preferably non-toxic to the recipient at the doses and concentrations used. The pharmaceutical composition of the present application includes, but is not limited to, a liquid, frozen or freeze-dried composition.

In the present application, the term "pharmaceutically acceptable carrier" typically includes a pharmaceutically acceptable carrier, excipient or stabilizer that is non-toxic at the dose and concentration used to a cell or mammal exposed thereto. Physiologically acceptable vectors may include, for example, buffers, antioxidants, polypeptides with low molecular weight (less than about 10 residues), proteins, hydrophilic polymers, amino acids, monosaccharides, disaccharides and other carbohydrates, chelating agents, sugar alcohols, salt-forming counter-ions such as sodium; and/or nonionic surfactants.

In the present application, the term "subject" generally refers to human or non-human animals, including, but not limited to, cats, dogs, horses, pigs, cows, sheep, rabbits, mice, rats, or monkeys.

In the present application, it is to be understood that the involved protein, polypeptide and/or amino acid sequence contains at least the following ranges: variants or homologs having the same or similar functions as the protein or polypeptide.

In the present application, the variant may be a protein or polypeptide that is subjected to substitution, deletion or addition with one or more amino acids in the amino acid sequence of the protein and/or the polypeptide (e.g., antibodies or fragments thereof that specifically bind to IL-11 protein). For example, the variant may contain a protein or polypeptide that has amino acid change caused by substitution, deletion, and/or insertion of at least one, e.g., 1-30, 1-20, or 1-10, yet e.g., 1, 2, 3, 4, or 5 amino acids. The functional variant may substantially maintain the biological properties of the protein or polypeptide prior to change (e.g., substitution, deletion, or addition). For example, the functional variant may maintain at least 60%, 70%, 80%, 90%, or 100% biological activity of the protein or polypeptide prior to change (e.g., the antigen binding ability). For example, the substitution can be a conservative substitution.

In the present application, the homologue may be a protein or polypeptide having at least about 85% (e.g., having at least about 85%, about 90%, about 91%, about 92%, about 93%, about 94%, about 95%, about 96%, about 97%, about 98%, about 99%, or more) sequence homology with the amino acid sequence of the protein and/or the polypeptide (e.g., the antibodies or fragments thereof that specifically bind to IL-11 protein).

In the present application, the homology generally refers to similarity, analogy, or association between two or more sequences. The "percentage of sequence homology" can be calculated by comparing two sequences to be aligned in a comparison window, determining the number of sites in which the same nucleic acid base (e.g., A, T, C, G, ad I) or the same amino acid residue (e.g., Ala, Pro, Ser, Thr, Gly, Val, Leu, Ile, Phe, Tyr, Trp, Lys, Arg, His, Asp, Glu, Asn, Gln, Cys and Met) are present in the two sequences to obtain the number of matching sites, dividing the number of matching sites by the total number of sites in the comparison window (i.e., window size), and multiplying the result by 100 to produce the percentage of sequence homology. Alignment to determine the percentage of sequence homology can be implemented in a variety of ways known in the art.

In the present application, the term "including" generally means to containing, generalizing, having or covering. In some cases, it also means "refer to", "composed of".

In the present application, the term "about" generally refers to a change in the range of 0.5% to 10% above or below a specified value, such as a change in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### DETAILED DESCRIPTION OF THE PRESENT INVENTION

### Isolated antigen-binding protein

In the present application, an isolated antigen-binding protein is provided and has one or more of the following properties: (1) the affinity to human and/or animal IL-11 is extremely high; (2) the activation of an IL-11-mediated hIL-11 Effector Reporter Cell can be blocked; and (3) fibrosis can be inhibited.

In the present application, the antigen-binding protein may include an antibody or an antigen-binding fragment thereof. In the present application, the antigen-binding fragment may include Fab, Fab', F(ab)₂, an Fv fragment, F(ab')₂, scFv, di-scFv, VHH, and/or dAb. In the present application, the antibody may include a monoclonal antibody, a chimeric antibody, a humanized antibody, and a fully human antibody.

### CDR

CDR of an antibody, also known as a complementarity-determining region, is part of a variable region. Amino acid residues in this region can be in contact with antigens or epitopes. The antibody CDR can be determined by a variety of encoding systems, such as CCG, Kabat, Chothia, IMGT, AbM, Kabat/Chothia integrated into account. These encoding systems are known in the art, and it specifically refers to, for example, http://www.bioinf.org.uk/abs/index.html#kabatnum. Those skilled in the art can use different encoding systems to determine the CDR region according to the sequence and structure of the antibody. Using different encoding systems, the CDR regions may differ. In the present application, the CDR encompasses CDR sequences divided according to any CDR division method; the CDR also encompasses variants thereof including amino acid sequences of the CDR after substitution, deletion and/or addition of one or more amino acids, For example, 1-30, 1-20 or 1-10, for another example, 1, 2, 3, 4, 5, 6, 7, 8 or 9 amino acid in substitution, deletion and/or insertion; it also covers homologs, which may be amino acid sequences that have at least about 85% (e.g., at least about 85%, 90%, 91%, 92%, 93%, 94%, 95%, 96%, 97%, 98%, 99% or more) sequence homology to the amino acid sequence of the CDR. In the present application, the isolated antigen-binding protein is defined by a Kabat encoding system.

In the present application, the isolated antigen-binding protein may contain at least one CDR in an antibody light chain variable region VH or VHH, and an amino acid sequence of the VH or VHH is as set forth in SEQ ID NO: 10.

In the present application, the isolated antigen-binding protein may contain HCDR3, and an amino acid sequence of the HCDR3 is as set forth in SEQ ID NO: 3.

In the present application, the isolated antigen-binding protein may contain HCDR2, and an amino acid sequence of the HCDR2 is as set forth in SEQ ID NO: 2.

In the present application, the isolated antigen-binding protein may contain HCDR1, and an amino acid sequence of the HCDR1 is as set forth in SEQ ID NO: 1.

In the present application, the isolated antigen-binding protein may contain HCDR3, HCDR2 and HCDR1; and the amino acid sequence of the HCDR3 is as set forth in SEQ ID NO: 3, the amino acid sequence of the HCDR2 is as set forth in SEQ ID NO: 2, and the amino acid sequence of the HCDR is as set forth in SEQ ID NO: 1.

### FR

In the present application, the antibody framework region FR refers to a part between more divergent (i.e., hypervariable) CDRs in the antibody variable region. Such framework regions are typically referred to as frameworks 1 to 4 (FR1, FR2, FR3, and FR4) and provide a framework for representing six CDRs (three from heavy chains and three from light chains) in a three-dimensional space to form antigen-binding surfaces.

In the present application, the isolated antigen-binding protein may contain H-FR1; and a C terminal of the H-FR1 is directly or indirectly linked to an N terminal of the HCDR1, and an amino acid sequence of the H-FR1 is as set forth in SEQ ID NO: 4.

In the present application, the isolated antigen-binding protein may contain H-FR2; and the H-FR2 is located between the HCDR1 and the HCDR2, and an amino acid sequence of the H-FR2 is as set forth in SEQ ID NO: 5.

In the present application, the isolated antigen-binding protein may contain H-FR3; and the H-FR3 is located between the HCDR2 and the HCDR3, and an amino acid sequence of the H-FR3 is as set forth in SEQ ID NO: 6.

In the present application, the isolated antigen-binding protein may contain H-FR4; and an N terminal of the H-FR4 is linked to a C terminal of the HCDR3, and an amino acid sequence of the H-FR4 is as set forth in SEQ ID NO: 7.

### VH/VHH

In the present application, the isolated antigen-binding protein may contain a variable region VH or VHH, and the amino acid sequence of the VH or VHH is as set forth in SEQ ID NO: 10.

In the present application, the isolated antigen-binding protein may contain an antibody or antigen-binding fragment thereof.

For example, the antigen-binding protein includes Fab, Fab', F(ab)₂, a Fv fragment, F(ab')₂, scFv, di-scFv, VHH and/or dAb.

For example, the antibody is selected from the group consisting of a monoclonal antibody, a chimeric antibody, a humanized antibody and a fully human antibody.

In the present application, the isolated antigen-binding protein may contain VHH, and the amino acid sequence of the VHH is as set forth in SEQ ID NO: 10.

In the present application, the isolated antigen-binding protein may contain an antibody heavy chain constant region. The antibody heavy chain constant region may be deviated from a human IgG heavy chain constant region. In some embodiments, the isolated antigen-binding protein may contain the antibody heavy chain constant region, and the antibody heavy chain constant region may be deviated from a human IgG1 heavy chain constant region. In some embodiments, the isolated antigen-binding protein may contain the antibody heavy chain constant region, and the antibody heavy chain constant region may be deviated from the human IgG heavy chain constant region and subjected to amino acid mutation.

For example, the amino acid sequence of the antibody heavy chain constant region is as set forth in SEQ ID NO: 8 or SEQ ID NO: 9.

In addition, it is to be noted that the isolated antigen-binding protein according to the present application may contain heavy chain and/or light chain sequences subjected to one or more conserved sequence modifications with the antigen-binding protein according to the present application. The "conserved sequence modifications" refer to amino acid modifications that do not significantly affect or change the antibody binding properties. Such conserved modifications include amino acid substitutions, additions, and deletions. The modification can be introduced into the isolated antigen-binding proteins according to the present application by standard techniques known in the art, such as point mutations and PCR-mediated mutations. The conserved amino acid substitution refers to the substitution of amino acid residues with amino acid residues having similar side chains. The group of amino acid residues having similar side chains is known in the art. These groups of amino acid residues include those with basic side chains (e.g., lysine, arginine, and histidine), acidic side chains (e.g., aspartic acid, and glutamic acid), polar uncharged side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine) and aromatic side chains (e.g., tyrosine, phenylalanine, tryptophan, and histidine). In some embodiments, one or more amino acid residues in the CDR region of the isolated antigen-binding protein according to the present application may be substituted with other amino acid residues of the same side chain group. Those skilled in the art know that some conserved sequence modifications do not cause antigen binding to disappear.

### Chimeric antigen receptor, polypeptide molecules, nucleic acid molecules, vector, cell, immunoconjugate, and pharmaceutical composition

In another aspect, the present application also provides a chimeric antigen receptor (CAR) that may contain a targeting domain binding to IL-11.

In another aspect, the present application provides polypeptide molecules which may contain the isolated antigen-binding protein according to the present application.

In some embodiments, the polypeptide molecules may contain a fusion protein. In some embodiments, the polypeptide molecules may be a fusion protein.

In another aspect, the present application provides isolated nucleic acid molecules which can encode the isolated antigen-binding protein according to the present application. For example, it can be produced or synthesized by: (1) amplification in vitro, such as by polymerase chain reaction (PCR) amplification; (2) cloning and recombination; (3) purification, e.g., fractionation by enzymatic digestion and gel electrophoresis; or (4) synthetic, e.g. chemical synthesis.

In another aspect, the present application provides a vector, which may contain the nucleic acid molecules according to the present application. In addition, the vector may further include other genes, for example, a marker gene that allows selection of the vector in an appropriate host cell and under an appropriate condition. In addition, the vector may further include an expression control element that allows correct expression of a coding region in an appropriate host. Such a control element is well known to those skilled in the art, and may be, for example, a promoter, ribosome binding site, enhancer, and other control elements that regulate gene transcription or mRNA translation. The vector may be transformed, transduced, or transfected into a host cell to express a genetic material element it carries within the host cell. The vector may be, for example, a plasmid, cosmid, virus, phage, or other vectors commonly used in, for example, genetic engineering. For example, the vector is an expression vector. In addition, the vector may also include components that facilitate to enter cells, such as viral particles, liposomes, or protein coats, but not just these.

In another aspect, the present application provides a cell, which may contain the nucleic acid molecules according to the present application or the vector according to the present application. In some embodiments, each type or each host cell may contain one or one type of the nucleic acid molecules or vector according to the present application. In some embodiments, each type or each host cell may contain a plurality of (e.g., 2 or more) or a plurality of types of (e.g., 2 or more) nucleic acid molecules or vectors according to the present application. For example, the vector according to the present application may be introduced into the host cell, such as eukaryotic cells, like cells from plants, fungi or yeast cells. In some embodiments, the cells can be bacterial cells (e.g., Escherichia coli), yeast cells, or other eukaryotic cells, such as COS cells, Chinese hamster ovary (CHO) cells, CHO-K1 cells, LNCAP cells, HeLa cells, 293T cells, COS-1 cells, SP2/0 cells, NS0 cells or myeloma cells. In some embodiments, the vector according to the present application can be introduced into the host cell through methods known in the art, such as electroporation, lipofectine transfection and lipofectamin transfection.

In another aspect, the present application provides an immunoconjugate, which may contain the isolated antigen-binding protein according to the present application.

In another aspect, the present application also provides a pharmaceutical composition, which may contain the isolated antigen-binding protein according to the present application, the polypeptide molecules according to the present application, the immunoconjugate according to the present application, the nucleic acid molecules according to the present application, the vector according to the present application and/or the cell according to the present application, and optionally a pharmaceutically acceptable carrier.

In some embodiments, the pharmaceutical composition may contain suitable preparations of one or more (pharmaceutically effective) carriers, stabilizers, excipients, diluents, solubilizers, surfactants, emulsifiers and/or preservatives. Acceptable ingredients of the composition are preferably non-toxic to the recipient at the doses and concentrations used. The pharmaceutical composition of the present application includes, but is not limited to, a liquid, frozen or freeze-dried composition.

In some embodiments, the pharmaceutical composition may be bispecific or multispecific molecules containing the isolated antigen-binding protein according to the present application. In some embodiments, in the bispecific or multispecific molecules, except the molecules that bind to IL-11, the target to which other molecules bind may be unrelated to IL-11.

In some embodiments, the pharmaceutical composition may also contain more than one active compound, which are generally the active compounds that have complementary activity and do not adversely affect each other. The type and effective amount of such drugs may depend on, for example, the amount and type of antagonists in the formulation, as well as the clinical parameters of the subject.

In some embodiments, the pharmaceutically acceptable carrier may include any and all solvents, dispersion media, coatings, isotonic agents, and absorption retardants that are compatible with drugs in administration and are generally safe, and non-toxic.

### Preparation method

In another aspect, the present application provides a method for preparing the antigen-binding protein. The method may include: the host cell as described in the present application is cultured under a condition that allows expression of the isolated antigen-binding protein. For example, appropriate media, appropriate temperatures and culture times are used; and these methods can be understood by those of ordinary skill in the art.

Any method suitable for the generation of the monoclonal antibodies can be used for generating the antigen-binding protein according to the present application. Appropriate immunization methods can be used, including adjuvants, immunostimulants, and repeat booster vaccinations, and one or more routes can be used. For example, a hybridoma preparation method can be used for acquiring splenocytes from immunized mice and fusing them with SP2/0 myeloma cells, and then hybridoma cell lines can be screened by HAT.

Any suitable form of IL-11 can be used as an immunogen (antigen) to produce non-human antibodies specific to IL-11, thus screening the biological activity of the antibody. For example, the initiated immunogen can be full-length mature human IL-11, including natural homodimers, or peptides containing single/multiple epitopes. The immunogen can be used alone or in combination with one or more immunogenicity enhancers known in the art.

The chimeric human antibody can be derived from any kind of immunoglobulins, including IgM, IgD, IgG, IgA, and IgE. In the present application, the antibody can be an IgG antibody, and an IgG1 isoform can be used. The antibodies can be screened by biological assays in the following embodiments to realize optimization of a necessary constant domain sequence, thus producing the required biological activity. Similarly, any kind of light chains can be used in the compounds and methods according to the present application. For example, κ chains or variants thereof may be used in the compounds and methods according to the present application.

### Method and use

In another aspect, the present application provides use of the isolated antigen-binding protein, the chimeric antigen receptor, the polypeptide molecules, the nucleic acid molecules, the vector, the cell, the immunoconjugate and/or the pharmaceutical composition in the manufacture of a medicament for preventing and/or treating diseases and/or disorders.

In another aspect, the present application provides a method for preventing and/or treating diseases and/or disorders, which may include: applying the isolated antigen-binding protein, the chimeric antigen receptor, the polypeptide molecules, the nucleic acid molecules, the vector, the cell, the immunoconjugate and/or the pharmaceutical composition according to the present application to a subject in need.

In another aspect, the isolated antigen-binding protein, the polypeptide molecules, the nucleic acid molecules, the vector, the cell, the immunoconjugate and/or the pharmaceutical composition according to the present application can be used for preventing and/or treating diseases and/or disorders.

In the present application, the diseases and/or disorders may be diseases and/or disorders associated to IL-11.

In the present application, the diseases and/or disorders may be inflammatory disease or tumor.

In the present application, the diseases and/or disorders include inflammatory diseases and/or tumor associated to IL-11.

In another aspect, the present application further provides a method for detecting IL-11 in a sample, which includes: applying the isolated antigen-binding protein, the polypeptide molecules, the nucleic acid molecules, the vector, the cell, the immunoconjugate and/or the pharmaceutical composition.

In some cases, the method for detecting IL-11 in the sample is an in vitro method. In some cases, the method for detecting IL-11 in samples is for non-therapeutic purposes. In some cases, the method for detecting IL-11 is not a diagnostic method.

In another aspect, the present application also provides an agent or a kit for detecting IL-11 in a sample, which contains the isolated antigen-binding protein, the polypeptide molecules, the nucleic acid molecules, the vector, the cell, the immunoconjugate and/or the pharmaceutical composition.

In another aspect, the present application provides use of the isolated antigen-binding protein, the polypeptide molecules, the nucleic acid molecules, the vector, the cell, the immunoconjugate and/or the pharmaceutical composition in preparation of a kit which is used for detecting presence and/or content of IL-11 in the sample.

Without intending to be limited by any theory, the following embodiments are only intended to illustrate the fusion protein, preparation method and use according to the present application, and are not intended to limit the scope of the present application.

### Examples

### Example 1 Preparation method of anti-human IL-11 alpaca monoclonal antibody-original antibody

Unimmunized alpacas were selected, 5 mL of blood was collected before immunization, and serum was isolated as a negative control. IL-11 Protein, Human, Recombinant (Sino Biological Inc., Cat No: 12225-HNCE) was used as antigens, and 0.5 mg of antigen was fully emulsified with an equal volume of complete Freund's adjuvant (Sigma, Cat No: F5881), and then injected subcutaneously at multiple points. Then, 0.25 mg of antigen and incomplete Freund's adjuvant (Sigma, Cat No.: F5506) were fully emulsified, and subcutaneously injected into the alpacas at multiple points at every 3 weeks. 1 week after the third and fourth immunizations, 50 mL of peripheral blood was collected for antibody titer titration and nanobody library construction. The serum after the third and fourth immunization was subjected to valence respectively, the antibody titer in serum after the third immunization was > 32000, the antibody titer in serum after the fourth immunization was > 64000, which both met the requirements of library construction.

### Example 2 Construction and screening of immune repertoire

The peripheral blood collected from the alpacas after the third and fourth immunization was isolated to obtain peripheral blood mononuclear cells (PBMCs); after combining, the total RNA was extracted and reversely transcribed into cDNA. An one-step PCR was carried to amplify VHH3+VHH4+VH-like VHH gene fragments. The VHH gene fragments were cloned into pShort phage plasmids, and a GA reaction product was electroporated into superstate cells SR320 containing helper phage, and phage was obtained after expanding cultivation, which was the immune repertoire. The quality data of the immune repertoire are shown in Table 1. The culture supernatant was collected to obtain an antibody library (Phage), which was sub-packed and stored at -80°C.

**Table 1 Statistics of Lib11-VHHall-related indexes in alpaca immune repertoire**

| Group | LB/Tet₁₀ (bacterial count) | LB/Carb₅₀ (Number of positive clones=Storage capacity) | GA recombination efficiency |
|---|---|---|---|
| VHHall | 2.2×10¹¹ | 2.5×10⁹ | 80% |

### Example 3 Biopanning and protein expression of antibody library

Through protein solid-phase screening, a Human IL-11 recombinant protein was used as a bait, and protein panning was carried out by multiple rounds to realize enrichment; and high-affinity positive clones were screened through phage ELISA. It was determined by monoclonal sequencing that 11 were unique, and the sequences were divided into 5 groups according to the difference of CDRH3 amino acid sequences. The phage enriched with Human IL-11 was subjected to NGS sequencing, and 20 sequences in NGS sequencing were transiently expressed by eukaryotic transientization, and 17 sequences were successfully expressed.

### Example 4 Preparation of 700068 heavy chain antibody

### 4.1 Preparation of 400208 plasmid (SEQ ID NO: 11)

A signal peptide sequence was added in front of an obtained antibody variable region gene for gene synthesis. A target gene was linked to a human FC containing mammalian cell expression vector through HindIII and BamHI digestion sites. Leucine L234 and Leucine L235 (EU numbering) in CH2 of Fc on the vector were mutated into Alanine A. The Fc mutations can eliminate the binding of antibody Fc to FcγR and C1q without affecting the binding to FcRn, thereby greatly attenuating the antibody ADCC/CDC effect. After linkage, a complete expression vector was obtained and numbered by 400208.

The 400208 expression vector was amplified by Escherichia coli, and a sufficient amount of plasmids was prepared through an endotoxin-free plasmid extraction kit (TIANGEN BIOTECH (BEIJING) CO., LTD., Article Number: DP117) and sequenced to confirm that the plasmids were completely consistent with theoretical sequences.

### 4.2 Antibody expression

The prepared plasmids were transiently transfected into host cells CHO-S and cultured in an incubator for 5-7 days. The supernatant of a cell culture medium was collected by centrifugation, and an antibody 700068 was obtained by purifying through a Protein A affinity column.

700068-VHH (SEQ ID NO: 10)

QVQLVESGGGLVQPGGSLRLSCAASGSIASIYTMAWYRQAPGKQRELVALSTNRGAT
DYADFVKGRFTISRDNAENTMYLQMNSLKPEDTAVYYCNGVASSWSTGFVASWGQGTQ
VTVSS

Note: The italicized underlined and bold part is the CDR area under the Kabat number.

700068-Fc (SEQ ID NO: 8)

GSEPKSCDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPG

### Example 5 Preparation of 700077 antibody

### 5.1 Preparation of 400231 plasmid (SEQ ID NO: 12)

Expression plasmids of the antibody 700068 were used as a template for PCR amplification to obtain a target gene fragment, and the PCR primers and sequences are shown in Table 1. A mammalian cell expression vector containing the human light chain constant region was digested with a restriction enzyme HindIII/EcoRI, and then linked to the target gene fragment. After linkage, a complete expression vector was obtained and numbered by 400231.

**Table 2 PCR primers and sequences**

| | |
|---|---|
| 400208-F(SEQ ID NO: 13) | GAGCAGCGAGCCCAAATCTTCTGACAAAACTCACACAT |
| 400208-R(SEQ ID NO: 14) | CAGAAGATTTGGGCTCGCTGCTCACGGTCACTTGG |
| 400216-F(SEQ ID NO: 15) | ACCGTCCTTGACACGAAGCTTGCCGCCACCATGGGATG |
| 400216-R(SEQ ID NO: 16) | GCTGATTATGATCAATGAATTCTCAACCCGGAGACAGGGAGAGGCTC |

A 400231 expression vector was amplified by Escherichia coli, and sufficient plasmids were prepared through a NucleoBond^{®} Xtra Maxi Plus EF plasmid extraction kit (MACHEREY-NAGEL, Article Number: 740426.50). The extracted plasmids were digested through a restriction enzyme pvuI (NEB, Article number: R0150L), and then precipitated with ethanol to obtain linearized plasmids, and the linearized plasmids were sequenced to confirm that the plasmids were completely consistent with the theoretical sequence.

### 5.2 Expression of 700077 antibody

The linearized plasmids 400231 were stably transfected into host cells CHO-K1 for expression; and after culturing for a period of time, the supernatant of the cell culture medium was collected by centrifugation; and the antibody 700077 was obtained by purifying through a Protein A affinity column.

| Name | Manufacturer | Article Number |
|---|---|---|
| Human IL-11, No Tag | Sino Biological | 12225-HNCE |
| Mouse IL-11, No Tag | Sino Biological | 50117-MNCE |
| HBS-EP+Buffer(10X) | Cytiva | BR-1006-69 |
| Series S Sensor Chip Protein A | Cytiva | 29-1275-56 |
| 10mM Glycine, pH1.5 | Cytiva | BR-1003-54 |
| BIACORE | Cytiva | T200 |

700077-VHH (SEQ ID NO: 10)

QVQLVESGGGLVQPGGSLRLSCAASGSIASIYTMAWYRQAPGKQRELVALSTNRGAT
DYADFVKGRFTISRDNAENTMYLQMNSLKPEDTAVYYCNGVASSWSTGFVASWGQGTQ
VTVSS

Note: The italicized underlined and bold part is the CDR area under the Kabat number.

700077-Fc (SEQ ID NO: 9)

EPKSSDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPG

### Example 6 Detection of affinity of 700068 heavy chain antibody with human and mouse IL-11

An SPR method was carried out to determine kinetic constants of human and mouse IL-11 binding to an antibody, and test materials and instruments were as follows:
The temperature of a sample storage and a flow cell of Biacore T200 was set to 25°C, a data collection frequency was 10 Hz. A Series S Protein A chip was used for capturing antibody samples to 300 RU as ligands, respectively. Human and mouse IL-11 antigens were diluted by a HBS-EP+ buffer solution with pH of 7.4 to reach a series of gradient concentrations of 50 nM, 25 nM, 12.5 nM, 6.25 nM, 3.125 nM, 1.56 nM, 0.78 nM, and 0.39 nM as analytes, and the HBS-EP+ buffer solution with pH of 7.4 was treated as 0 concentrations for background deduction. A multi-cycle kinetic method was carried out, the analytic flow rate was set to be 30 µL/min, the binding time was 120 s, the dissociation time was 600 s, and 10 mM of glycine (pH: 1.5) was adopted to regenerate at 50 µL/min for 60s. 1: 1 binding mode was adopted, Fit Local Kinetics mode was used for analyzing data.

The experimental results are shown in Table 3:
The results of the affinity constants (KD/M) with a variety of genera show that the affinity of the anti-IL-11 monoclonal heavy chain antibody 700068 according to the present invention with human and mouse IL-11 is 10⁻⁰⁹M orders of magnitude, and the anti-IL-11 monoclonal heavy chain antibody 700068 has an extremely strong affinity with human and mice antigens.

**Table 3 Results of affinity of 700068 heavy chain antibody with human and mouse IL-11**

| Sample | Antigen | ka (1/Ms) | kd (1/s) | KD (M) |
|---|---|---|---|---|
| 700068 | Human IL-11 | 6.89×10⁵ | 1.33×10⁻³ | 1.93×10⁻⁰⁹ |
| | Mouse IL-11 | 4.87×10⁵ | 1.07×10⁻³ | 2.20×10⁻⁰⁹ |

| | | | | |
|---|---|---|---|---|
| Note: NB refers to non-binding | | | | |

### Example 7 Detection of affinity of 700068 heavy chain antibody to rat IL-11

A BLI method was carried out to determine the kinetic constant of binding of IL-11 with antibodies in rats, and test materials and instruments were as follows:

| Name | Manufacturer | Article Number |
|---|---|---|
| Recombinant Rat Interleukin-11(IL-11)), hFc Tag | CusaBio | CSB-MP859561RAd9 |
| PBS | BasalMedia | B320KJ |
| Polysorbate 20 | Sigma-Aldrich | 44112 |
| SA Sensor | Sartorius | 18-5021 |
| Chromalink biotin354(sulfo NHS) | Vector Laboratories | B-9007-105K |
| Octet | Sartorius | RH96 |

An SA sensor was adopted to load 5 µg/mL of biotinylated sample as Ligand and a Rat IL-11 solution diluted by gradient as Analyte for kinetic detection. Temperature was 30°C, Shaking Rate was 1000rpm, Association was 360s, Dissociation was 360s, and 1:1 Binding Model Global Fit was utilized to analyze the kinetic constants.

The experimental results are shown in Table 4:
The results of the affinity constants (KD/M) with a variety of genera show that the affinity of the humanized anti-IL-11 monoclonal antibody 700068 according to the present invention with human and rat IL-11 is 10⁻⁰⁹M orders of magnitude, and the humanized anti-IL-11 monoclonal antibody 700068 has an extremely strong affinity with rat antigens.

**Table 4 Results of affinity of 700068 heavy chain antibody with rat IL-11**

| Sample | Antigen | R² | ka (1/Ms) | kd (1/s) | KD (M) |
|---|---|---|---|---|---|
| 700068 | Rat IL-11 | 1.000 | 4.749×10⁴ | 5.566×10⁻⁰⁵ | 1.172×10⁻⁰⁹ |

### Example 8 Detection of affinity of 700068 monoclonal heavy chain antibody with monkey IL-11

An SPR method was carried out to determine the kinetic constant of binding of monkey antigen-antibody, and test materials and instruments were as follows:

| Name | Manufacturer | Article Number |
|---|---|---|
| Cynomolgus IL-11, No Tag | Sino Biological Inc. | 90925-CNCE |
| Amine-coupling kits | Cytiva | BR-1000-50 |
| HBS-EP+ Buffer(10X) | Cytiva | BR-1006-69 |
| Series S Sensor Chip CM5 | Cytiva | BR-1005-30 |
| 10mM Glycine, pH1.5 | Cytiva | BR-1003-54 |
| BIACORE | Cytiva | Biacore 8K |

The temperature of a sample storage and a flow cell of Biacore 8k was set to 25°C, a data collection frequency was 10 Hz. An Amine Coupling Kit was used for performing amine-coupling to fix Cynomolgus IL-11 as ligands on a Series S Sensor Chip CM5. Monkey IL-11 was diluted by a HBS-EP+ buffer solution with pH of 7.4 to reach a series of gradient concentrations of 20 nM, 10 nM, 5 nM, 2.5 nM and 1.25 nM as analytes, and the HBS-EP+ buffer solution with pH of 7.4 was treated as 0 concentrations for background deduction. A single-cycle kinetic method was carried out, the analytic flow rate was set to be 30 µL/min, the binding time was 120 s, the dissociation time was 600 s, and 10 mM of glycine (pH: 1.5) was adopted to regenerate at 50 µL/min for 60s. 1:1 binding mode was adopted, Fit Local Kinetics mode was used for analyzing data.

The experimental results are shown in Table 5:
The results of the affinity constants (KD/M) show that the affinity of the anti-IL-11 heavy chain antibody 700068 according to the present invention with monkey IL-11 is 10⁻⁰⁹M orders of magnitude, and the anti-IL-11 monoclonal heavy chain antibody 700068 has an extremely strong affinity with monkey antigens.

**Table 5 Results of affinity of 700068 heavy chain antibody with monkey IL-11**

| Sample | Antigen | ka (1/Ms) | kd (1/s) | KD (M) |
|---|---|---|---|---|
| 700068 | Cynomolgus IL-11 | 4.85×10⁵ | 2.21×10⁻⁰³ | 4.56×10⁻⁰⁹ |

### Example 9 Non-specific binding test of 700068 monoclonal heavy chain antibody

The temperature of a sample storage and a flow cell of Biacore 8k was set to 25°C, and Lysozyme solution from chicken egg and Trypsin inhibitor type 1-S from Glycine max were subjected to amine coupling on a CM5 chip as ligands. Antibodies were diluted to 1 µM, Flow Rate was 10 µL/min, Association was 900s, Dissociation was 1200s, 0.85% phosphoric acid was used for regenerating for 30 s at a flow rate of 50 µL/min, and then 50 Mm of sodium hydroxide was used for regenerating for 30 s at a flow rate of 50 µL/min. Binding stability signals were collected to analyze the non-specific electrostatic binding effect of the antibodies.

The results of quality control of Polyclonal rabbit anti-lysozyme and Anti-trypsin inhibitor antibody for Lysozyme solution from chicken egg and Trypsin inhibitor type 1-S from Glycine max show that it can be determined that egg white lysozyme and soybean trypsin inhibitor have normal activity after amine coupling to fix to the CM5 chip, that is, the fixed ligand activity remains good throughout the experiment. The PI of Trypsin inhibitor type 1-S from Glycine max was 4.5 and that of Lysozyme was 11.3, and in an HBS-EP buffer system with pH of 7.4, the Trypsin inhibitor type 1-S from Glycine max had a strong negative charge, and the Lysozyme had a strong positive charge.

The experimental results are shown in Table 6:
The binding response values of all the test samples to Lysozyme and Trypsin are than 20RU, which may indicate that the test samples do not have obvious non-specific electrostatic binding.

**Table 6 Non-specific binding data of 700068 heavy chain antibody**

| Antibody | Lysozyme (RU) | Trypsin inhibitor (RU) | Deactived Carboxymethyl Dextran(RU) | Carboxymethyl Dextran(RU) |
|---|---|---|---|---|
| Buffer solution | 0 | 0 | 0 | 0 |
| 700068 | -17.4 | 5.2 | -1.7 | -1.4 |

### Example 10 Blocking of anti-IL-11 monoclonal heavy chain antibody (700068) to activation of IL-11-mediated hIL-11 Effector Reporter Cell

HIL-11 Effector Reporter Cell cells (COBIOER BIOSCIENCES, Cat#:CBP74114) were collected, and resuspended with a DMEM medium (Gibco, Cat#:11965092) containing 10% FBS (Gibco, Cat#: 10099141C); the cells were diluted to 1.2×10⁶ cells/mL, and added to a 96-well white plate (Corning, Cat#: 3599) by 50 µL per well; and the 96-well white plate was placed in an incubator containing 5% CO₂ and at temperature of 37°C for culturing overnight. The anti-IL-11 antibody was diluted to 120 µg/mL with the DMEM medium containing 10% FBS, and then diluted by a 3-fold gradient to reach 10 concentrations, 11 concentration gradients in total. Antibodies with different concentration gradients were added to the 96-well white plate by 25 µL per well. Human IL-11 (novoprotein, Cat#:C006) was diluted to 1 ng/mL through the DMEM0 medium containing 10% FBS, and was added to the above 96-well white plate by 25 µL per well. The 96-well white plate was placed in an incubator containing 5% CO₂ and at temperature of 37°C for culturing for 6 h. After the incubation, the 96-well white plate was taken out, and balanced at room temperature for 15 min. A Bright-Glo agent (Promega, Cat#: E2620) was added by 100 µL per well, and a microplate reader (MD SpectraMax^{®} i3x) was used for full-wavelength fluorescence detection.

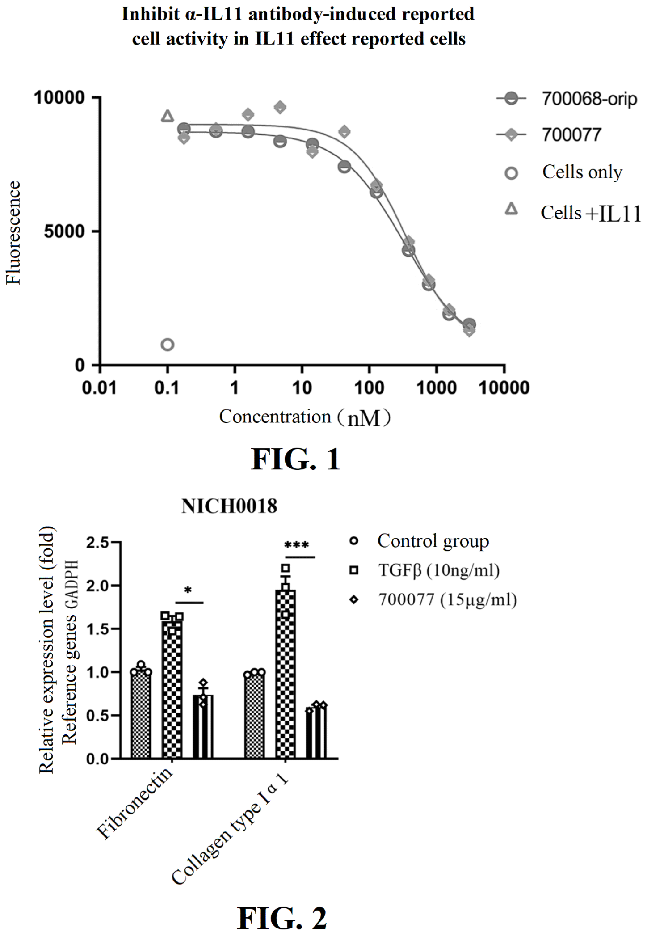

The experimental results are shown in FIG. 1:
The anti-IL-11 heavy chain antibody 700068 according to the present invention can block the activation of the IL-11-mediated hIL-11 Effector Reporter Cell.

### Example 11 In vitro fibrosis inhibition ability of IL-11 heavy chain antibody

The ability of an anti-IL-11 heavy chain antibody 700077 in inhibiting TGFβ (transforming growth factor β)-induced fibrosis in NICH0018 (skin fibroblasts of healthy humans) was detected. After NICH0018 cells in the logarithmic growth phase in a culture flask absorbed a medium (89% DMEM (Dulbecchia medium) + 10% FBS (fetal bovine serum) + 1% P/S (penicillin, streptomycin)), the NICH0018 cells were washed twice with 10 mL of PBS; and after 2 mL of 0.25% trypsin was added, the NICH0018 cells were digested in an incubator (containing 5% carbon dioxide, 37 °C,) for 3-5 min. After adding to medium to terminate digestion, a cell suspension was obtained; and the cell suspension was centrifuged (500g×5min) to remove a culture solution, resuspended with medium to reach 1 mL, and then counted through a counter. The cells were placed into a six-well plate by 5×10⁵ cells per well; the medium was replenished to 2 mL per well; and after culturing for 8 h in the incubator and attaching to wall, a complete medium (DMEM without FBS) was replaced and starved for 16 h. The medium was prepared according to a final concentration of 10 ng/ml of TGFβ or 15 µg/ml of 700077; and after the original medium was absorbed, 2 mL of normal medium was added according to the blank group, 2 mL of 10 ng/ml TGFβ medium was added to a stimulation group, and 2 mL of medium with a final concentration of 10 ng/ml of TGFβ and 15 µg/ml of 700077 was added to the antibody group, with three replicates in each group. After 24 h of incubation in the incubator, the supernatant was collected (stored at - 20°C for subsequent ELISA detection), and washed twice with PBS; and the cells were collected into RNase-free EP tubes through a cell scraper and centrifuged (500 g×5 min) to obtain a cell pellet.

RT-PCR was used for detecting the expression of fibrosis-related genes FN (encoding fibronectin) and COL1A1 (encoding type I collagen alpha1) in cells. 350 µL of Buffer RLT was added to the cell pellet and mixed with the cells to achieve homogeneous lysis, and 350 µL of 70% ethanol was added to the lysate and mixed evenly. The previous lysate was transferred to a 2 mL collection tube with the RNeasy MinElute spin column, and centrifuged at 8,000 g for 15 s; and the filtrate was removed. 350 µL of Buffer RW1 was added to the RNeasy MinElute spin column, and centrifuged at 8,000 g for 15 s; and the filtrate was removed. The RNeasy MinElute spin column was placed into a new 2 mL collection tube, and 500 µL of Buffer RPE was added, and centrifuged at 8,000 g for 15 s; and the filtrate was removed. 500 µL of 80% ethanol was added to the RNeasy MinElute spin column, and centrifuged at 8,000 g for 2 min; and the collection tube was removed. The RNeasy MinElute spin column was placed into a new 2 mL collection tube, and centrifuged through a centrifuge with a lid opened at the highest speed for 5 min; and the filtrate and collection tube were removed. The RNeasy MinElute spin column was placed into a new 1.5 mL collection tube, 14 µL of RNase-free water was added dropwise to the center of the absorption column in a suspension manner, and centrifuged through the centrifuge at the highest speed for 1 min; and the RNA concentration was detected after elution of RNA.

The genome removal reaction system and reaction conditions are as follows:

| Name of agent | Volume (µL) |
|---|---|
| 5X gDNA Eraser Buffer | 2 µL |
| gDNA Eraser | 1 µL |
| Total RNA | 250 ng |
| RNase Free dH₂O | up to 10µL |

| Temperature(°C) | Time |
|---|---|
| 42 | 2 min |
| 4 | ∞ |

After obtaining a reaction solution with genome removed, a reverse transcription reaction was carried out, and the reaction system and reaction conditions were as follows:

| Name of agent | Volume (µL) |
|---|---|
| Reaction solution with genome removed | 10 µL |
| PrimeScript RT Enzyme Mix I | 1 µL |
| RT Primer Mix | 1 µL |
| 5xPrimerScript Buffer 2(for Real Time) | 4 µL |
| RNase Free dH₂O | 4 µL |

| Temperature(°C) | Time |
|---|---|
| 37 | 15 min |
| 85 | 5 s |
| 4 | ∞ |

Quantitative PCR was performed after cDNA was obtained:
The reaction solution was prepared according to the table below, and the mixture was added to the corresponding reaction tube, 11.2 µL each. 2 µL of cDNA and 6.8 µL of DEPC H₂O were mixed into a Mix and added to the corresponding wells. Meanwhile, negative control wells were set up, and the template was sterile water.

| Agent | Volume |
|---|---|
| 2×Fast SYBE Green Master Mix | 10 µL |
| Forward Primer (10 µM) | 0.6 µL |
| Reverse Primer (10 µM) | 0.6 µL |

Program Settings: a comparative CT (ΔΔCT) method was adopted, and a software running program was set up. PCR amplification procedure: denaturing was carried out at 95°C for 10 min, and was performed by 1 cycle; denaturation was carried out at 95°C for 15 s, extension was carried out at 60°C for 30 s, and the operation was performed by 40 cycles; denaturation was carried out at 95°C for 15 s, extension was carried out at 60°C for 1 min, and the gradient was increased to 95°C from 0.15°C. Quantitative PCR was performed.

The experimental results are shown in FIG. 2:
The anti-IL-11 heavy chain antibody 700077 according to the present invention can inhibit the increase of FN and COL1A1 induced by TGFβ.

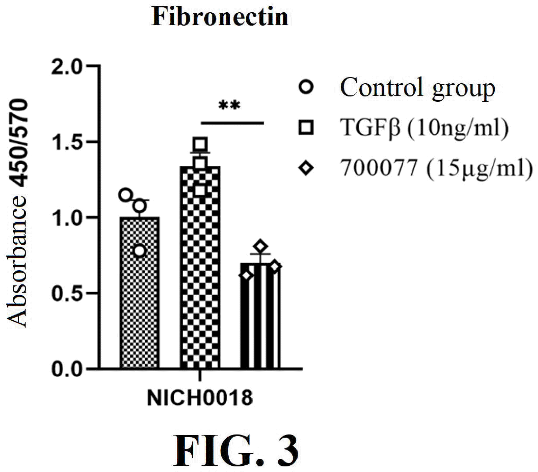

The experimental results are shown in FIG. 3:
The results of FN gene expression are verified at the protein level by ELISA (enzyme-linked immunosorbent assay), and it demonstrates that 700077 can inhibit TGFβ-induced fibrosis in the NICH0018 cells.

Note: In FIG. 2 and FIG. 3, the average value of the data is expressed as ±SEM, n=3. *P≤0.05, ***P≤0.001, and one-way analysis of variance was adopted.

## Claims

1. An isolated antigen-binding protein, targeting IL-11 and comprising HCDR3, HCDR2 and HCDR1 of a heavy chain variable region VH or VHH, wherein an amino acid sequence of the HCDR3 is as set forth in SEQ ID NO: 3, an amino acid sequence of the HCDR2 is as set forth in SEQ ID NO: 2, and an amino acid sequence of the HCDR1 is as set forth in SEQ ID NO: 1.

2. The antigen-binding protein according to claim 1, comprising H-FR1, wherein a C terminal of the H-FR1 is directly or indirectly linked to an N terminal of the HCDR1, and an amino acid sequence of the H-FR1 is as set forth in SEQ ID NO: 4.

3. The antigen-binding protein according to any one of claims 1 to 2, comprising H-FR2, wherein the H-FR2 is located between the HCDR1 and the HCDR2, and an amino acid sequence of the H-FR2 is as set forth in SEQ ID NO: 5.

4. The antigen-binding protein according to any one of claims 1 to 3, comprising H-FR3, wherein the H-FR3 is located between the HCDR2 and the HCDR3, and an amino acid sequence of the H-FR3 is as set forth in SEQ ID NO: 6.

5. The antigen-binding protein according to any one of claims 1 to 4, comprising H-FR4, wherein an N terminal of the H-FR4 is linked to a C terminal of the HCDR3, and an amino acid sequence of the H-FR4 is as set forth in SEQ ID NO: 7.

6. The antigen-binding protein according to any one of claims 1 to 5, comprising an antibody heavy chain variable region VH, wherein an amino acid sequence of the VH is as set forth in SEQ ID NO: 10.

7. The antigen-binding protein according to any one of claims 1 to 6, comprising an antibody or an antigen-binding fragment thereof.

8. The antigen-binding protein according to claim 7, wherein the antigen-binding fragment comprises Fab, Fab', F(ab)₂, a Fv fragment, F(ab')₂, scFv, di-scFv, VHH and/or dAb.

9. The antigen-binding protein according to any one of claims 7 to 8, wherein the antigen-binding fragment is VHH.

10. The antigen-binding protein according to any one of claims 1 to 9, wherein an amino acid sequence of the VHH is as set forth in SEQ ID NO: 10.

11. The antigen-binding fragment according to any one of claims 7 to 10, wherein the antibody is selected from the group consisting of a monoclonal antibody, a chimeric antibody, a humanized antibody, a fully human antibody, a single-chain antibody, a nanobody and/or a heavy chain antibody.

12. The antigen-binding protein according to claim 11, wherein the antibody is a nanobody and/or a heavy chain antibody.

13. The antigen-binding protein according to any one of claims 1 to 12, comprising an antibody heavy chain constant region.

14. The antigen-binding protein according to any one of claims 1 to 13, wherein the antibody heavy chain constant region is derived from a human IgG heavy chain constant region.

15. The antigen-binding protein according to any one of claims 1 to 14, wherein the antibody heavy chain constant region is derived from a human IgG1 heavy chain constant region.

16. The antigen-binding protein according to any one of claims 1 to 15, wherein an amino acid sequence of the antibody heavy chain constant region is as set forth in SEQ ID NO: 8 or SEQ ID NO: 9.

17. A chimeric antigen receptor, comprising a targeting domain, wherein targeting domain comprises the antigen-binding protein according to any one of claims 1 to 16.

18. Polypeptide, comprising the isolated antigen-binding protein according to any one of claims 1 to 16 or the chimeric antigen receptor according to claim 16.

19. An immunoconjugate, comprising the isolated antigen-binding protein according to any one of claims 1 to 16.

20. One or more isolated nucleic acid molecules, encoding the isolated antigen-binding protein according to any one of claims 1 to 16, the chimeric antigen receptor according to claim 17 or polypeptide molecules according to claim 18.

21. A vector, comprising the nucleic acid molecules according to claim 20.

22. A cell, comprising the isolated antigen-binding protein according to any one of claims 1 to 16, the chimeric antigen receptor according to claim 17, the polypeptide molecules according to claim 18, the immunoconjugate according to claim 19, the nucleic acid molecules according to claim 20 or the vector according to claim 21.

23. A pharmaceutical composition, comprising the isolated antigen-binding protein according to any one of claims 1 to 16, the chimeric antigen receptor according to claim 17, the polypeptide molecules according to claim 18, the immunoconjugate according to claim 19, the nucleic acid molecules according to claim 20, the vector according to claim 21 and/or the cell according to claim 22, and optionally a pharmaceutically acceptable carrier.

24. A method for preparing the isolated antigen-binding protein according to any one of claims 1 to 16, comprising: culturing the cell according to claim 22, under conditions that allow expression of the antigen-binding protein.

25. Use of the isolated antigen-binding protein according to any one of claims 1 to 16, the chimeric antigen receptor according to claim 17, the polypeptide molecules according to claim 18, the immunoconjugate according to claim 19, the nucleic acid molecules according to claim 20, the vector according to claim 21, the cell according to claim 22 and/or the pharmaceutical composition according to claim 23 in the manufacture of a medicament for preventing, alleviating and/or treating diseases and/or disorders.

26. The use according to claim 25, wherein the diseases and/or disorders comprise diseases associated to IL-11.

27. The use according to claim 25 or 26, wherein the diseases and/or disorders comprise inflammatory disease or tumor.

28. A method for detecting IL-11 in a sample, comprising: applying the isolated antigen-binding protein according to any one of claims 1 to 16, the chimeric antigen receptor according to claim 17, the polypeptide molecules according to claim 18, the immunoconjugate according to claim 19, the nucleic acid molecules according to claim 20, the vector according to claim 21, the cell according to claim 22 and/or the pharmaceutical composition according to claim 23.

29. An agent or a kit for detecting IL-11 in a sample, comprising the isolated antigen-binding protein according to any one of claims 1 to 16, the chimeric antigen receptor according to claim 17, the polypeptide molecules according to claim 18, the immunoconjugate according to claim 19, the nucleic acid molecules according to claim 20, the vector according to claim 21, the cell according to claim 22 and/or the pharmaceutical composition according to claim 23.
